# EUROPEAN PATENT SPECIFICATION

(11) **EP 3 915 119 B1**
(45) Date of publication and mention of the grant of the patent: **15.05.2024**
(21) Application number: 20788279.6
(22) Date of filing: 08.04.2020
(51) Int. Cl.: H04L 9/40, G06F 21/31, G06F 21/62, G16H 10/60, H04L 9/08, H04L 9/32, H04L 67/06, A61B 5/1171, A61B 5/1172, A61B 5/00, G16H 10/65

(54) **ELECTRONIC DEVICE AND METHOD FOR SHARING MEDICAL INFORMATION BY ELECTRONIC DEVICE**
ELEKTRONISCHE VORRICHTUNG UND VERFAHREN ZUR GEMEINSAMEN NUTZUNG MEDIZINISCHER INFORMATIONEN DURCH DIE ELEKTRONISCHE VORRICHTUNG
DISPOSITIF ÉLECTRONIQUE ET PROCÉDÉ DE PARTAGE D'INFORMATIONS MÉDICALES PAR UN DISPOSITIF ÉLECTRONIQUE

(30) Priority: 11.04.2019 KR 20190042677
(43) Date of publication of application: 01.12.2021
(73) Proprietor: Samsung Electronics Co., Ltd., Suwon-si, Gyeonggi-do 16677 (KR)
(72) Inventor: KIM, Joohyun, Suwon-si Gyeonggi-do 16677 (KR)
(74) Representative: Gulde & Partner
(86) International application number: PCT/KR2020/004741
(87) International publication number: WO 2020/209596

(56) References cited:
- WO-A1-2016/123359
- US-A1- 2016 232 318
- US-A1- 2017 262 603

## Description

### Technical Field

Various embodiments of the disclosure relate to an electronic device and a method for sharing medical information.

### Background Art

Sharing medical information is disclosed e.g. in US 2017/0262603 A1 and US 2016/023218 A1. Medical information may include information related to medical activities, such as the entire record of hospital treatments of a user, activities according to the prescription of a doctor after medical treatments of the user, or activities that the user performs for disease prevention and health care. For example, medical information may include electric medical record (EMR) data or health care data.

Medical information may be stored and managed in an institution associated with medical care, such as a hospital or a pharmacy, or may be stored and managed in a designated medical information management institution. In order to view medical information, a user may visit a medical institution or a medical information management institution, respectively, in an offline manner, and may view and collect his/her medical information.

The above information is presented as background information only to assist with an understanding of the disclosure. No determination has been made, and no assertion is made, as to whether any of the above might be applicable as prior art with regard to the disclosure.

### Disclosure of Invention

### Technical Problem

Recently, there is a need for a technology that enables viewing and collecting of a user's medical information online. In other words, there is a demand for a technology that enables a user to access a medical institution server online, authenticate himself/herself, and view or download digitized medical information by means of an electronic device without visiting a medical institution. A medical institution may not disclose or share a user's medical information arbitrarily without consent of the user for security of the medical information. It may be cumbersome for a user to visit a medical institution, obtain a document, which records medical information, offline, and deliver the document directly to a third party (e.g., another person or another medical institution) in order to provide the medical information to the third party.

It may be convenient if a user can collect his/her medical information online and transfer the medical information to a third party. However, viewing, downloading, or collecting medical information, and providing or delivering the collected medical information directly to a third party online by a user may be vulnerable in security and may be legally restricted. Further, in order to provide medical information of a user to a third party other than the user, even a medical institution must obtain the user's consent for each piece of medical information to be provided and ensure that the provided medical information is not leaked to a person or institution to which disclosure of the medical information is not allowed. Therefore, an additional technique may be required to provide medical information online.

Even if medical information may be provided to a third party while being secured online with the consent of a user, due to the huge amount and type of medical information, it is not easy for a medical institution to specify medical information to be provided, an object to be provided with the medical information, and a period for providing the medical information.

Aspects of the disclosure are to address at least the above-mentioned problems and/or disadvantages and to provide at least the advantages described below. Accordingly, an aspect of the disclosure is to provide an electronic device that allows a user to collect and store his/her medical information online and share the stored medical information with a third party via an external electronic device, and a method for sharing medical information by the electronic device.

Another aspect of the disclosure is to provide an electronic device that allows a user to select medical information to be provided, from his/her medical information, an object to be provided with the medical information, and a period for providing the medical information, and to share the selected medical information with a third party via an external electronic device, and a method for sharing medical information by the electronic device.

Another aspect of the disclosure is to provide an electronic device that allows a user to provide his/her medical information to a specified external electronic device, receive, from the external electronic device, access information (e.g., endpoint information or Internet protocol (IP) address information) for accessing the medical information of the user, and transfer the received access information to a third party, so as to share the medical information of the user with the third party, and a method for sharing medical information by the electronic device.

Additional aspects will be set forth in part in the description which follows and, in part, will be apparent from the description, or may be learned by practice of the presented embodiments.

### Solution to Problem

In accordance with an aspect of the invention, an electronic device according to claim 1 is provided. The electronic device includes a communication circuit, a memory configured to store instructions, and at least one processor, wherein the instructions are configured, when executed, to cause at least one processor to, obtain medical information from a medical information providing device, encrypt the medical information, obtain information of at least one recipient allowed to receive the encrypted medical information, the recipient being a user of a second electronic device, transmit, to an external electronic device, the encrypted medical information and information of the at least one recipient using the communication circuit, obtain, from the external electronic device, access information for accessing the encrypted medical information, and provide the obtained access information to the second electronic device.

In accordance with an aspect of the disclosure, which is not part of but useful for the understanding of the claimed invention, an electronic device is provided. The electronic device includes a transceiver, a storage configured to store instructions, and at least one processor, wherein the instructions are configured, when executed, to cause the at least one processor to, receive a request to share medical information, from a first electronic device via the transceiver, receive encrypted medical information from the first electronic device via the transceiver, store the encrypted medical information, and obtain access information for accessing the encrypted medical information, and provide the access information to the first electronic device.

In accordance with an aspect of the invention, a method according to claim 11 and a non-transitory computer-readable recording medium according to claim 12 and having recorded thereon at least one program comprising commands is provided. The non-transitory computer-readable recording medium, when executed by a computer, performs the method comprising obtaining medical information from a medical information providing device, encrypting the medical information, obtaining information of at least one recipient allowed to receive the encrypted medical information, the recipient being a user of a second electronic device, transmitting, to an external electronic device, the encrypted medical information and information of the at least one recipient using a communication circuit, obtaining, from the external electronic device, access information for accessing the encrypted medical information, and providing the acquired obtained information to the second electronic device.

### Advantageous Effects

According to various embodiments, a user can collect and store his/her medical information online, and can share the stored medical information with a third party via an external electronic device.

According to various embodiments, a user can select medical information to be provided, from his/her medical information, an object to be provided with the medical information, and a period for providing the medical information, and can share the selected medical information with a third party via an external electronic device.

According to various embodiments, a user can provide his/her medical information to a specified external electronic device, receive, from the external electronic device, access information (e.g., endpoint information or IP address information) for accessing the medical information of the user, and transfer the received access information to a third party, so as to share the medical information of the user with the third party.

According to various embodiments, a user can collect his/her medical information from a medical institution, can post his/her collected medical information in an isolated location, and can personally share the posted medical information directly with a third party to allow access. Further, the user can also update or stop sharing his/her shared medial information.

According to various embodiments, a medical institution that is a third party can receive location information, in which personal medical information is stored, directly from an individual, so as to acquire medical information by using the location information, and can use the acquired medical information in medical activities.

According to various embodiments, a method of collecting and sharing data by an individual directly from various medical institutions can eliminate an existing situation in which it is difficult for a medical institution to collect specific medical information of a specific user, which is distributed in various other medical institutions. In particular, a medical institution that collects data shares the data via a centralized relay device, e.g., an external electronic device, instead of directly accessing an electronic device of a user, so as to enable stable access to medical information.

Other aspects, advantages, and salient features of the disclosure will become apparent to those skilled in the art from the following detailed description, which, taken in conjunction with the annexed drawings, discloses various embodiments of the disclosure.

### Brief Description of Drawings

The above and other aspects, features, and advantages of certain embodiments of the disclosure will be more apparent from the following description taken in conjunction with the accompanying drawings, in which:
FIG. 1 is a diagram illustrating a medical information sharing system according to an embodiment of the disclosure;
FIG. 2 is a diagram illustrating a configuration of a first electronic device according to an embodiment of the disclosure;
FIG. 3 is a diagram illustrating a configuration of an external electronic device according to an embodiment of the disclosure;
FIG. 4 is a diagram illustrating an operation of sharing medical information with a second electronic device by a first electronic device via an external electronic device according to an embodiment of the disclosure;
FIG. 5 is a diagram illustrating an operation of acquiring medical information shared by a first electronic device, from an external electronic device by a second electronic device according to an embodiment of the disclosure;
FIG. 6 is a block diagram of an electronic device in a network environment according to an embodiment of the disclosure;
FIG. 7 is a flowchart illustrating a method of sharing medical information of an electronic device according to an embodiment of the disclosure;
FIG. 8 is an example of a user interface (UI) for sharing medical information of an electronic device according to an embodiment of the disclosure;
FIG. 9 is an example of a screen for groups subject to sharing of medical information according to an embodiment of the disclosure;
FIG. 10 is an example illustrating a screen for, when medical information is being shared, the medical information that is being shared, according to an embodiment of the disclosure;
FIG. 11 is a diagram illustrating an example of data representing medical information sharing condition configuration information according to an embodiment of the disclosure;
FIG. 12 is an example of an authentication message according to an embodiment of the disclosure; and
FIG. 13 is an example of a screen for a user authentication scheme configuration according to an embodiment of the disclosure.

Throughout the drawings, like reference numerals will be understood to refer to like parts, components, and structures.

### Mode for the Invention

The following description with reference to the accompanying drawings is provided to assist in a comprehensive understanding of various embodiments of the disclosure as defined by the claims. It includes various specific details to assist in that understanding but these are to be regarded as merely exemplary. Descriptions of well-known functions and constructions may be omitted for clarity and conciseness.

The following description of various embodiments of the disclosure is provided for illustration purpose.

It is to be understood that the singular forms "a," "an," and "the" include plural referents unless the context clearly dictates otherwise. Thus, for example, reference to "a component surface" includes reference to one or more of such surfaces.

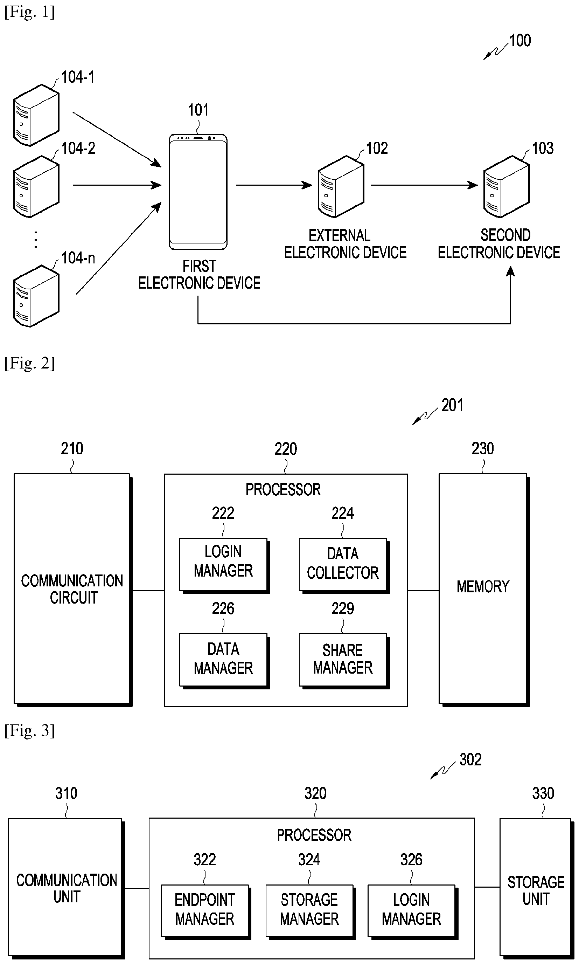

FIG. 1 is a diagram illustrating a medical information sharing system according to an embodiment of the disclosure.

Referring to FIG. 1, a medical information sharing system 100 according to various embodiments may include at least one medical information providing device 104-1 to 104-n, a first electronic device 101, an external electronic device 102, and a second electronic device 103.

The at least one medical information providing device 104-1 to 104-n (hereinafter, also referred to as "electronic medical record (EMR) provider") according to various embodiments may be a device of each medical-related institution. According to various embodiments, the at least one medical information providing device 104-1 to 104-n may include one of a hospital server, a pharmacy server, or a health care center server, and may further include servers of various institutions related to medical activities. According to various embodiments, the at least one medical information providing device 104-1 to 104-n may store and manage medical information associated with a user, and may provide medical information to an authenticated user after user authentication. For example, the hospital server may store medical information corresponding to a user's hospital treatment procedure. The pharmacy server may store medical information corresponding to information of medicine purchased by a user or information of medicine prescribed to the user. The health care center server may store medical information corresponding to various activities performed by a user for disease prevention and health care, for example, exercise activities, therapeutic activities, or medical check-up activities. In addition, servers of various institutions related to medial activities may provide various medical information related to health and medical care of a user. According to various embodiments, medical information may include electric medical record (EMR) data or health care data. For example, medical information may include fast healthcare interoperability resources (FHIR)-based data or medical data of various schemes. According to various embodiments, a unit of medical information may be a record.

The first electronic device 101 (hereinafter, also referred to as "EMR collector") according to various embodiments may be various types of devices. According to various embodiments, the first electronic device 101 may include one of a portable communication device (e.g., a mobile phone or a smartphone), a computer device, a portable multimedia device, a portable medical device, a camera, a wearable device, or a home appliance. The first electronic device 101 according to various embodiments may not be limited to the aforementioned devices. The first electronic device 101 according to various embodiments may request, after user authentication, medical information of a user from each of the medical information providing devices 104-1 to 104-n, and may temporarily or periodically receive medical information from each of the medical information providing devices 104-1 to 104-n. According to various embodiments, medical information may include electric medical record (EMR) data or health care data. For example, medical information may include fast healthcare interoperability resources (FHIR)-based data or medical data of various schemes. According to various embodiments, the first electronic device 101 may store and manage the received medical information. According to an embodiment, the first electronic device 101 may encrypt and store the received medical information. According to various embodiments, the first electronic device 101 may encrypt and store the medical information in a designated encryption scheme. According to various embodiments, the designated (or preconfigured) encryption scheme may be an encryption scheme selected from among various encryption schemes. According to various embodiments, the encryption scheme may be an encryption scheme using a symmetric key or an asymmetric key. For example, the encryption scheme may be one of a Rivest Shamir Adleman(RSA) scheme, a data encryption standard (DES) scheme, or an advanced encryption standard(AES) scheme using an encryption key. According to various embodiments, the encryption scheme may use a scheme of using an encryption key, wherein the encryption key is generated with a random number, or generated using personal biometric information, such as voice, iris, face, or vein of a recipient user. According to various embodiments, the encryption scheme may use a scheme of generating, as an ID, a part of a result of encrypting the encryption key, generating, as a password, the other part of the result of encrypting the encryption key, and then enabling decryption to be performed using values input as the ID and the password.

According to various embodiments, the first electronic device 101 may include a normal storage area and a secured storage area, and may store the encrypted medical information in the secured storage area. According to various embodiments, the first electronic device 101 may share the encrypted medical information with a third party (e.g., the second electronic device 103) via the external electronic device 102. According to various embodiments, the first electronic device 101 may configure a recipient (e.g., a sharing target for sharing of medical information) allowed to receive the encrypted medical information or a sharing condition of the encryption medical information. According to various embodiments, the first electronic device 101 may designate, as a recipient, a contact selected by a user from at least one pre-stored contact, may designate, as a recipient, at least one contact input by the user, or may designate, as a recipient, one of recipients automatically acquired by the first electronic device 101. For example, the contact may be one of various types, such as a phone number, an email address, an account, and the like. According to various embodiments, the first electronic device 101 may designate, as sharing condition configuration information of the encrypted medical information, at least one of a sharing (or access) authority, a sharing range, a sharing period, a sharing frequency, an authentication scheme, an authentication validity period, an encryption scheme, and an encryption key of the encrypted medical information. According to various embodiments, the sharing authority may be an authority allowed to receive the encrypted medical information. According to various embodiments, the sharing range may be an item allowed to be shared among items included in the encrypted medical information. According to various embodiments, the sharing period may be a period in which the encrypted medical information may be shared. According to various embodiments, the sharing frequency may be the number of times that the encrypted medical information may be viewed or received. According to various embodiments, the authentication scheme may be a user authentication scheme for a recipient. According to various embodiments, the authentication validity period may be a user authentication validity period. According to various embodiments, the encryption scheme may be an encryption scheme of the medical information. According to various embodiments, the encryption key may be encryption key information used to decrypt the medical information. According to various embodiments, the first electronic device 101 may further configure various conditions associated with sharing of the encrypted medical information.

According to various embodiments, the first electronic device 101 may provide the external electronic device 102 with the encrypted medical information, or may provide the external electronic device 102 with the encrypted medical information and information of the recipient allowed to receive the encrypted medical information. According to various embodiments, the first electronic device 101 may further provide the external electronic device 102 with the sharing condition configuration information of the encrypted medical information. According to various embodiments, the first electronic device 101 may provide the medical information to the external electronic device 102 in a designated unit (e.g., record).

According to various embodiments, the first electronic device 101 may receive updated medical information from each of the medical information providing devices 104-1 to 104-n, and when the updated medical information is received, the first electronic device 101 may encrypt the updated medical information and provide the same to the external electronic device 102. According to various embodiments, the first electronic device 101 may provide the encrypted medical information, and then may receive, from the external electronic device 102, access information for accessing the encrypted medical information. For example, the access information may include address information (e.g., an end point address or an IP address) in which the encrypted medical information is stored, or a uniform resource locator (URL) enabling reception of the encrypted medical information. According to various embodiments, the first electronic device 101 may provide the access information for accessing the encrypted medical information to the recipient allowed to receive the encrypted medical information. According to various embodiments, the first electronic device 101 may further transfer decryption information for decryption of the encrypted medical information, in addition to the access information. For example, the decryption information may include an encryption key, a password, and the like. According to various embodiments, the encrypted medical information may be provided in a scheme different from that of providing the access information separately from providing of the access information.

According to various embodiments, the external electronic device 102 (e.g., also referred to as an "EMR sharing server") may be a designated server. According to various embodiments, the external electronic device 102 may be a server designated by a user or a server designated by a public institution (or a medical institution). The external electronic device 102 may receive the medical information (or the encrypted medical information), may receive the encrypted medical information and information of a recipient allowed to receive the medical information, may receive the encrypted medical information and sharing condition configuration information of the medical information, or may receive the encrypted medical information, information of a recipient allowed to receive the medical information, and sharing condition configuration information of the medical information, from the first electronic device 101. According to various embodiments, the external electronic device 102 may store the encrypted medical information received from the first electronic device 101, and may generate access information for accessing the encrypted medical information. For example, the access information may include address information (e.g., an end point information or an IP address information) in which the encrypted medical information is stored, or a uniform resource locator (URL) enabling reception of the encrypted medical information. According to various embodiments, the external electronic device 102 may update the access information when updated encrypted medical information is received from the first electronic device 101. For example, if updated second medical information is received while medical information is being shared (posted), the external electronic device 102 may generate and share new access information. According to various embodiments, when the updated encrypted medical information is received from the first electronic device 101, the external electronic device 102 may use the previous access information as it is, and may update only medical information associated with the previous access information. For example, if the updated second medical information is received while the medical information is being shared (posted), the external electronic device 102 may replace the medical information with the second medical information while maintaining the access information.

According to various embodiments, the external electronic device 102 may provide access information of the encrypted medical information to the first electronic device 101, and the access information may be transferred to the second electronic device 103. According to various embodiments, the external electronic device 102 may receive a medical information request using the access information from the second electronic device 103. According to various embodiments, the external electronic device 102 may authenticate a user of the second electronic device 103 having requested the medical information. For example, the external electronic device 102 may request an ID and a password from the second electronic device 103, may receive the ID and the password from the second electronic device 103, and may authenticate the user of the second electronic device 103. According to various embodiments, various schemes may be used for authenticating the user of the second electronic device 103. For example, various authentication schemes, such as a random text scheme, a biometric(fingerprint, iris, face, voice, vein, or the like) recognition scheme, a voice recognition scheme, and an OAuth recognition scheme, may be selectively used for authenticating the user of the second electronic device 103. According to various embodiments, the random text scheme may be a scheme of authenticating a recipient by using a random text, such as a random password, etc. If random text, to which a recipient is designated, is input, authentication of a user of the recipient may be successful. A fingerprint recognition scheme may be a scheme of authenticating a fingerprint of a recipient. An iris recognition scheme may be a scheme of authenticating an iris of a recipient. A face recognition scheme may be a scheme of authenticating a recipient by using a stored photo (e.g., in a gallery) or an image of the recipient, which is obtained via a camera. If a registered image of the recipient and an image received from a recipient user are the same, user authentication of the recipient may be successful. A voice authentication scheme may be a scheme of authenticating the recipient by using a voice file corresponding to a voice of the recipient. If the voice file and the voice received from the recipient user are the same, user authentication of the recipient may be successful. Other authentication schemes may be selectively used in addition thereto.

According to various embodiments, if user authentication for the user of the second electronic device 103 is successful, the external electronic device 102 may provide the encrypted medical information to the second electronic device 103. According to various embodiments, if user authentication for the user of the second electronic device 103 is successful, the external electronic device 102 may provide the encrypted medical information to the second electronic device 103, or may request decryption information, decrypt the encrypted medical information by using the decryption information, and then provide unencrypted medical information. For the decryption information, information enabling decryption according to an encryption scheme may be provided. According to various embodiments, the encryption scheme may be an encryption scheme using a symmetric key or an asymmetric key. For example, the encryption scheme may be one of a Rivest Shamir Adleman(RSA) scheme, a data encryption standard (DES) scheme, or an advanced encryption standard(AES) scheme using an encryption key. According to various embodiments, the encryption scheme may use a scheme of using an encryption key, wherein the encryption key is generated with a random number, or generated using personal biometric information, such as voice, iris, face, or vein of a recipient user. According to various embodiments, the encryption scheme may use a scheme of generating, as an ID, a part of a result of encrypting the encryption key, generating, as a password, the other part of the result of encrypting the encryption key, and then enabling decryption to be performed using values input as the ID and the password.

According to various embodiments, the external electronic device 102 may receive an encrypted medical information request using access information, from each of a plurality of electronic devices including the second electronic device 103. According to various embodiments, the external electronic device 102 may store a history of providing medical information (or encrypted medical information). For example, the external electronic device 102 may store which medical information has been provided to whom and when. According to various embodiments, the external electronic device 102 may provide the history of providing medical information to the first electronic device 101. According to various embodiments, the first electronic device 101 may display, on a display, the history of providing the medical information. For example, the first electronic device 101 may provide the history of providing the medical information according to each frequency or each date, such as a reference count or reference data.

According to various embodiments, the external electronic device 102 may store, for a designated period, medical information (or encrypted medical information) received from the electronic device 101, or may delete the medical information (or the encrypted medical information) after the designated period. According to various embodiments, the external electronic device 102 may activate access information for accessing the medical information (or the encrypted medical information) such that the access information is available for the designated period, and may deactivate the access information such that the access information is unavailable after the designated period.

According to various embodiments, the second electronic device 103 (for example, also referred to as "EMR consumer") may be various types of devices. According to various embodiments, the second electronic device 103 may include one of a portable communication device (e.g., a mobile phone or a smartphone), a computer device, a portable multimedia device, a portable medical device, a camera, a wearable device, or a home appliance, or may include a device of a medical related institution. For example, a device of a medical institution may include one of a hospital server, a pharmacy server, an insurance company server, or a health care center server, and may further include servers of various institutions related to medical activities. The second electronic device 103 according to various embodiments may not be limited to the aforementioned devices. According to various embodiments, the second electronic device 103 may receive, from the first electronic device 101, the access information for accessing the medical information. For example, the second electronic device 103 may receive address information (e.g., end point information or an IP address) in which the medical information is stored, or a uniform resource locator (URL) enabling reception of the medical information, by using a text message (e.g., a short message service), an email, or the like via communication. According to various embodiments, the second electronic device 103 may transmit a medical information request to the external electronic device 102 by using access information, and may enable a user of the second electronic device 103 to be authenticated from the external electronic device 102. If user authentication is successful, the second electronic device 103 may receive and use the encrypted medical information from the external electronic device 102. According to various embodiments, the second electronic device 103 may further receive, from the first electronic device 101, decryption information in addition to the access information. According to various embodiments, the second electronic device 103 may provide the decryption information to the external electronic device 102 so as to receive decrypted medical information, or may acquire medical information by using the decryption information when the encrypted medical information is received. According to various embodiments, the second electronic device 103 may receive the decryption information in a scheme different from that of the access information.

FIG. 2 is a diagram illustrating a configuration of a first electronic device according to an embodiment of the disclosure.

Referring to FIG. 2, a first electronic device 201 (e.g., the first electronic device 101 of FIG. 1) may include a communication circuit 210, a processor 220, and a memory 230.

According to various embodiments, the communication circuit 210 may establish a wired or wireless communication channel between the first electronic device 201 and at least one medical information providing device (e.g., the medical information providing devices 104-1 to 104-n of FIG. 1), between the first electronic device 201 and an external electronic device (the external electronic device 102 of FIG. 1), and between the first electronic device 201 and a second electronic device (e.g., the second electronic device 103 of FIG. 1), and/or may support communication via the established communication channel. The communication circuit 210 may include one or more communication processors which are operated independently of the processor 220 (e.g., an application processor) and support wired communication or wireless communication).

According to various embodiments, the processor 220 may include one or more processors. For example, the processor 220 may include a login manager 222, a data collector 224, a data manager 226, or a share manager 229. Each of the login manager 222, the data collector 224, the data manager 226, and the share manager 229 may be a separate processor or may be a module in the processor 220, wherein each module may be configured by a combination of one or more, or two or more thereof.

According to various embodiments, the login manager 222 may login to each of at least one information providing device (at least one medical information providing device 104-1 to 104-n of FIG. 1) via the communication circuit 210, and may perform user authentication associated with the first electronic device 201.

According to various embodiments, the data collector 224 may temporarily or periodically receive medical information (or data corresponding to medical information) from at least one medical information providing device (at least one medical information providing device 104-1 to 104-n of FIG. 1).

According to various embodiments, the data manager 226 may store the received medical information in the memory 230, and may manage the medical information stored in the memory 230. According to various embodiments, the data manager 226 may encrypt the received medical information, and may store the encrypted medical information in the memory 230.

According to various embodiments, the share manager 229 may share the stored medical information (or the encrypted medical information) with others (e.g., the second electronic device 103 of FIG. 2). According to various embodiments, the share manager 229 may receive and store a recipient (e.g., a sharing target for sharing of the medical information) allowed to receive the medical information or sharing condition configuration information of the medical information. According to various embodiments, the recipient allowed to receive the medical information may be a contact selected from among at least one contact stored in the memory 230, and may be a contact automatically acquired from the outside via the communication circuit. According to various embodiments, the sharing condition configuration information of the medical information may include at least one among a sharing (or access) authority, a sharing range, a sharing period, a sharing frequency, an authentication scheme, an authentication validity period, an encryption scheme, and an encryption key of the encrypted medical information. For example, the sharing authority may be an authority allowed to receive the encrypted medical information, the sharing range may be an item allowed to be shared among items included in the encrypted medical information, the sharing period may be a period in which the encrypted medical information may be shared, the sharing frequency may be the number of times that the encrypted medical information may be viewed or received, the authentication scheme may be a user authentication scheme for a recipient, the authentication validity period may be a validity period after user authentication, the encryption scheme may be an encryption scheme of the medical information, and the encryption key may be encryption key information used to decrypt the medical information. According to various embodiments, in addition to the above conditions, various conditions associated with sharing the encrypted medical information may be further included.

According to various embodiments, the share manager 229 may provide medical information (or encrypted medical information) to an external electronic device (e.g., the external electronic device 102 of FIG. 1), may provide the external electronic device 102 with medical information (or encrypted medical information) and information of a recipient allowed to receive the medical information, may provide the external electronic device 102 with medical information (e.g., encrypted medical information) and sharing condition configuration information of the medical information, or may provide the external electronic device 102 with medical information (e.g., encrypted medical information), information of a recipient allowed to receive the medical information, and sharing condition configuration information of the medical information. According to various embodiments, the share manager 229 may provide the medical information (or the encrypted medical information) to the external electronic device 102, and then may receive access information for accessing the medical information (or the encrypted medical information) from the external electronic device 102. For example, the access information may include address information (e.g., an end point information or an IP address information) in which the medical information (or encrypted medical information) is stored, or a uniform resource locator (URL) enabling reception of the medical information (or the encrypted medical information). According to various embodiments, the share manager 229 may provide the access information for accessing the medical information to the recipient allowed to receive the medical information. According to various embodiments, when the encrypted medical information is provided, the share manager 229 may further transfer decryption information for decryption of the encrypted medical information, in addition to the access information. For example, the decryption information may include an encryption key, a password, and the like.

According to various embodiments, the processor 220 may include a secure operating system that operates separately from a normal operating system that performs overall control of the first electronic device 201, and may include the login manager 222, the data collector 224, the data manager 226, or the share manager 229 via the secure operating system. The processor 220 may perform operations associated with the login manager 222, the data collector 224, the data manager 226, or the share manager 229. For example, the secure operating system may include a secure operating system associated with Knox or Secure World in the Trust zone.

The memory 230 may store various data used by at least one element (e.g., the processor 220 or the communication circuit 210) of the first electronic device 201. The data may include, for example, software, and input data or output data for a command related to the software. The memory 230 may include a volatile memory or a nonvolatile memory. A program may be stored as software in the memory 230, and may include, for example, instructions that enable the login manager 222, the data collector 224, the data manager 226, or the share manager 229 to operate. According to various embodiments, the memory 230 may include a normal storage area and a secured storage area, and medical information (or encrypted medical information) may be stored in the secured storage area.

According to various embodiments, an electronic device (e.g., the first electronic device 101 of FIG. 1 or the first electronic device 201 of FIG. 2) may include a communication circuit ( e.g., the communication circuit 210 of FIG. 2), a memory (e.g., the memory 230 of FIG. 2) configured to store instructions, and at least one processor (e.g., the processor 220 of FIG. 2), wherein the instructions are configured, when executed, to cause the at least one processor to: acquire medical information; encrypt the medical information; acquire information of at least one recipient allowed to receive the encrypted medical information; transmit, to an external electronic device, the encrypted medical information and information of the at least one recipient by means of the communication circuit; acquire, from the external electronic device, access information for accessing the encrypted medical information; and provide the acquired access information to the at least one recipient.

According to various embodiments, the access information may include address information in which the encrypted medical information is stored.

According to various embodiments, the access information may include a URL enabling reception of the encrypted medical information.

According to various embodiments, the access information may be provided to at least one recipient via one of various message transmission schemes, for example, a short messaging service (SMS), a multimedia messaging service (MMS), email, or the like.

According to various embodiments, the electronic device (e.g., the first electronic device 101 of FIG. 1 or the first electronic device 201 of FIG. 2) may further include an instruction causing the at least one processor to provide decryption information for decryption of the encrypted medical information to the at least one recipient.

According to various embodiments, the electronic device (e.g., the first electronic device 101 of FIG. 1 or the first electronic device 201 of FIG. 2) may further include an instruction causing the at least one processor to configure a sharing condition including at least one of an authority, a sharing range, a sharing period, a sharing frequency, a user authentication scheme, an authentication validity period, an encryption scheme, or an encryption key, which are associated with the medical information.

According to various embodiments, the electronic device (e.g., the first electronic device 101 of FIG. 1 or the first electronic device 201 of FIG. 2) may further include an instruction configured to cause the at least one processor to provide the sharing condition associated with the medical information to the external electronic device.

According to various embodiments, the electronic device (e.g., the first electronic device 101 of FIG. 1 or the first electronic device 201 of FIG. 2) may further include a display, and may further include an instruction causing the at least one processor to enable the display to display a first screen for configuration of the sharing condition.

According to various embodiments, the electronic device (e.g., the first electronic device 101 of FIG. 1 or the first electronic device 201 of FIG. 2) may further include an instruction causing the at least one processor to enable the display to display a second screen for selection of the user authentication scheme.

According to various embodiments, the user authentication scheme may include at least one of a random text scheme, an iris recognition scheme, a fingerprint recognition scheme, a face recognition scheme, a voice recognition scheme, and a vein recognition scheme.

According to various embodiments, the electronic device (e.g., the first electronic device 101 of FIG. 1 or the first electronic device 201 of FIG. 2) may include a normal storage area and a secured storage area, and the medical information or the encrypted medical information may be stored in the secured storage area (e.g., a trust zone).

FIG. 3 is a diagram illustrating a configuration of an external electronic device according to an embodiment of the disclosure.

Referring to FIG. 3, an external electronic device 302 (e.g., the external electronic device 102 of FIG. 1) may include a communication unit 310 (e.g., a transceiver), a processor 320, and a storage unit 330 (e.g., a memory).

According to various embodiments, the communication unit 310 may establish a wired or wireless communication channel between the external electronic device 302 and a first electronic device (e.g., the first electronic device 101 of FIG. 1 or the first electronic device 201 of FIG. 2) or between the external electronic device 302 and a second electronic device (e.g., the second electronic device 103 of FIG. 1), and may support communication via the established communication channel. The communication unit 310 may include one or more communication processors which are operated independently of the processor 320 and support wired communication or wireless communication).

According to various embodiments, the processor 320 may include one or more processors. For example, the processor 320 may include an endpoint manager 322, a storage manager 324, or a login manager 326. Each of the endpoint manager 322, the storage manager 324, and the login manager 326 may be a separate processor, or may be a module in the processor 320.

According to various embodiments, the endpoint manager 322 may generate and manage access information (endpoint) enabling access to medical information (or encrypted medical information) received from the first electronic device (e.g., the first electronic device 101 of FIG. 1 or the first electronic device 201 of FIG. 2). According to various embodiments, the endpoint manager 322 may receive multiple pieces of medical information from multiple respective electronic devices, and may generate and manage multiple pieces of access information corresponding to the received multiple pieces of medical information, respectively. For example, the access information may include address information (e.g., an end point information or an IP address information) in which the medical information (or encrypted medical information) is stored, or a uniform resource locator (URL) enabling reception of the medical information (or the encrypted medical information). According to various embodiments, the endpoint manager 322 may activate access information for accessing the medical information (or the encrypted medical information) such that the access information is available for a designated period, and may deactivate the access information such that the access information is unavailable after the designated period.

According to various embodiments, the storage manager 324 may store the medical information (or the encrypted medical information) received from the first electronic device (e.g., the first electronic device 101 of FIG. 1 or the first electronic device 201 of FIG. 2) in the storage unit 330. For example, when multiple pieces of medical information are received from multiple respective electronic devices, the storage manager 324 may classify a storage area for each electronic device, and may store medical information corresponding to each electronic device in the storage area for each electronic device. According to various embodiments, the storage manager 324 may associate and store medical information (or encrypted medical information) and information of a recipient allowed to receive the medical information, may associate and store medical information (or encrypted medical information) and sharing condition configuration information of the medical information, or may associate and store medical information (or encrypted medical information), information of a recipient allowed to receive the medical information, and sharing condition configuration information of the medical information. According to various embodiments, when updated medical information (or encrypted medical information) is received, the storage manager 324 may update medical information (or encrypted medical information) stored in the storage unit 330.

According to various embodiments, if a medical information request using the access information is received from the second electronic device (e.g., the second electronic device 103 of FIG. 1) via the communication unit 310, the login manager 326 may perform user authentication for the second electronic device 103. According to various embodiments, the login manager 326 may request an ID and a password from the second electronic device 103 having requested the medical information, may receive the ID and the password from the second electronic device 103, and may authenticate a user of the second electronic device 103. According to various embodiments, if user authentication for the second electronic device 103 is successful, the login manager 326 may provide the medical information (or the encrypted medical information) to the second electronic device 103. According to various embodiments, if user authentication for the second electronic device 103 is successful, the login manager 326 may provide the encrypted medical information to the second electronic device 103, or may request decryption information, decrypt the encrypted medical information by using the decryption information, and then provide unencrypted medical information. According to various embodiments, the login manager 326 may include an authentication manager (OAuth manager) and/or a configuration manager. The OAuth manager may perform user authentication, and the configuration manager may check sharing condition configuration information of the medical information, so as to allow the medical information based on the sharing condition configuration information to be provided.

According to various embodiments, the storage unit 330 may store medical information (or encrypted medical information) received from the first electronic device (e.g., the first electronic device 101 of FIG. 1 or the first electronic device 201 of FIG. 2). According to various embodiments, the storage unit 330 may store a history of providing the medical information (or the encrypted medical information). For example, the storage unit 330 may store which medical information has been provided to whom and when. According to various embodiments, according to control of the storage manager 324, the storage unit 330 may store, for a designated period, the medical information (or the encrypted medical information) received from the first electronic device 101, or the medical information (or the encrypted medical information) may be deleted after the designated period.

According to various embodiments, the electronic device (e.g., the external electronic device 102 of FIG. 1 or the external electronic device 302 of FIG. 3) may include a communication unit (e.g., the communication unit 310 of FIG. 3), a storage unit (e.g., the storage unit 330 of FIG. 3) configured to store instructions, and at least one processor (e.g., the processor 320 of FIG. 3), wherein the instructions are configured, when executed, to cause the at least one processor to: receive a request to share medical information, from a first electronic device via the communication unit; receive encrypted medical information from the first electronic device via the communication unit; store the encrypted medical information, and acquire access information for accessing to the encrypted medical information; and provide the access information to the first electronic device.

According to various embodiments, the access information may include address information in which the encrypted medical information is stored.

According to various embodiments, the access information may include a uniform resource locator (URL) enabling reception of the encrypted medical information.

According to various embodiments, the electronic device (e.g., the external electronic device 102 of FIG. 1 or the external electronic device 302 of FIG. 3) may further include instructions which cause: a sharing request using the access information to be received from a second electronic device; a user of the second electronic device to be authenticated on the basis of the sharing request; and when user authentication for the second electronic device succeeds, the encrypted medical information to be provided.

According to various embodiments, the electronic device (e.g., the first electronic device 101 of FIG. 1 or the external electronic device 302 of FIG. 3) may further include an instruction configured to receive a sharing condition associated with the medical information from the first electronic device.

According to various embodiments, the sharing condition associated with the medical information may include at least one of a sharing authority, a sharing range, a sharing period, a sharing frequency, a user authentication scheme, an authentication validity period, an encryption scheme, or an encryption key.

According to various embodiments, the electronic device (e.g., the external electronic device 102 of FIG. 1 or the external electronic device 302 of FIG. 3) may further include an instruction that configures access corresponding to the medical information to be unavailable if the sharing period expires.

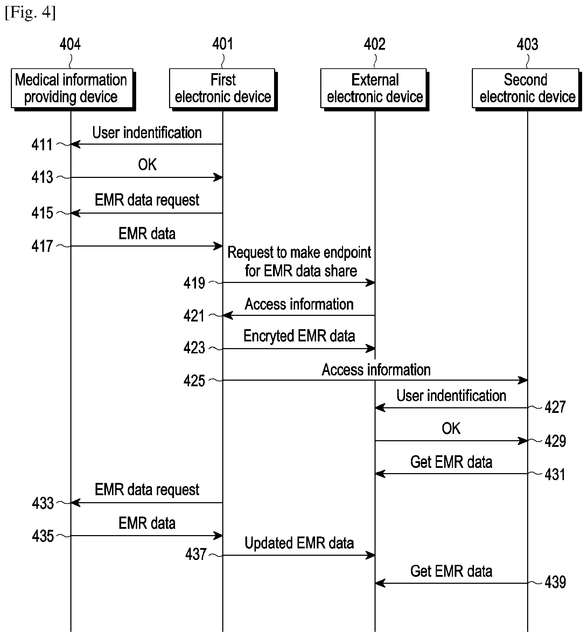

FIG. 4 is a diagram illustrating an operation of sharing medical information with a second electronic device by a first electronic device via an external electronic device according to an embodiment of the disclosure.

Referring to FIG. 4, in operation 411, a first electronic device 401 (e.g., the first electronic device 101 of FIG. 1 or the first electronic device 201 of FIG. 2) according to various embodiments may request user identification from a medical information providing device 404 (e.g., one of at least one medical information providing device 11-15 of FIG. 1).

In operation 413, the medical information providing device 404 may perform user authentication for the first electronic device 401, and may transmit information (OK) indicating success of the authentication when the user authentication succeeds. For example, the medical information providing device 404 may receive an identification (ID) and a password from the first electronic device 401, may perform user authentication using the received ID and password, and may transmit information indicating the success of the authentication.

In operation 415, if the authentication succeeds, the first electronic device 401 may request its own medical information from the medical information providing device 404 (EMR data request). According to various embodiments, the first electronic device 401 may request its own medical information once or multiple times, or may request its own medical information temporarily or periodically.

In operation 417, the medical information providing device 404 provides medical information (EMR data) to the first electronic device 401 in response to a medical information request. According to various embodiments, the medical information providing device 404 may encrypt the medical information (EMR data), and may provide the encrypted medical information (EMR data) to the first electronic device 401.

In operation 419, the first electronic device 401 may request an external electronic device 402 to generate an endpoint for sharing of medical information of a first user (request to make endpoint for EMR data share). The external electronic device 402 may generate an endpoint for access to the medical information, and may acquire access information for accessing the endpoint. According to various embodiments, the endpoint may be a storage area in which the medical information (or the encrypted medical information) is stored. For example, the endpoint may be a secured storage area. According to various embodiments, the access information for accessing the endpoint may include address information (e.g., endpoint information or IP address information) in which the encrypted medical information is stored, or a uniform resource locator (URL) enabling reception of the encrypted medical information.

In operation 421, the external electronic device 402 may provide the first electronic device 401 with access information for access to the endpoint in which the medical information is to be stored.

In operation 423, the first electronic device 401 may provide the medical information (EMR data) (or the encrypted medical information (encrypted EMR data)) to the external electronic device 402. According to various embodiments, the first electronic device 401 may provide, in addition to the encrypted medical information, recipient information associated with the encrypted medical information and sharing condition configuration information associated with the encrypted medical information to the external electronic device 402. According to various embodiments, the encrypted medical information may be stored (posted) in the endpoint of the external electronic device 402.

In operation 425, the first electronic device 401 provides a second electronic device 403 with access information enabling access to the endpoint. The second electronic device 403 may request to access the endpoint of the external electronic device 402 by using the access information.

In operation 427, the second electronic device 403 may request user authentication (user identification) from the external electronic device 402 for access to the endpoint. According to various embodiments, the external electronic device 402 may authenticate whether a user of the second electronic device 403 is a user included in the recipient information associated with the medical information. According to various embodiments, the external electronic device 402 may authenticate whether the user of the second electronic device 403 is a user satisfying the sharing condition configuration information associated with the medical information.

In operation 429, the external electronic device 402 may perform user authentication for the second electronic device 403, and may transmit information (OK) indicating success of the authentication when the user authentication succeeds. For example, the external electronic device 402 may receive an identification (ID) and a password from the second electronic device 403, may perform user authentication for the second electronic device 403 by using the received ID and password, and may transmit information indicating the success of the authentication.

In operation 431, the second electronic device 403 may receive the encrypted medical information from the endpoint of the external electronic device 402 by using the access information (get EMR data).

In operation 433, the first electronic device 401 may request second medical information (EMR data request). According to various embodiments, the first electronic device 401 may request the second medical information if a specified frequency or period arrives after the medical information is requested or if there is an input for requesting updated second medical information from a user.

In operation 435, the medical information providing device 404 may provide the second medical information (EMR data) to the first electronic device 401 in response to the request for the second medical information. According to various embodiments, the medical information providing device 404 may encrypt the second medical information (EMR data), and may provide the encrypted second medical information (EMR data) to the first electronic device 401.

In operation 437, the first electronic device 401 may provide the second medical information (updated EMR data) to the external electronic device 402. The external electronic device 402 may update medical information that has been stored (or posted) in the previously generated endpoint. For example, the external electronic device 402 may delete the medical information and store (or post) the second medical information, or may store (or post) the second medical information in addition to the medical information.

In operation 439, the second electronic device 403 may receive the updated medical information (e.g., the encrypted second medical information) from the endpoint of the external electronic device 402 by using the access information (get EMR data).

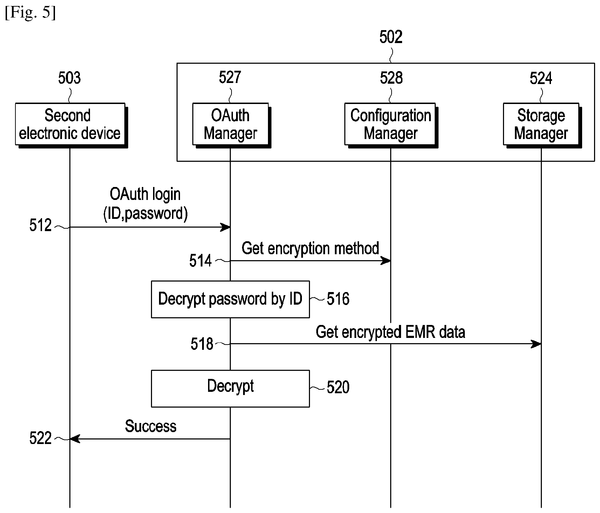

FIG. 5 is a diagram illustrating an operation of acquiring medical information shared by a first electronic device, from an external electronic device by a second electronic device according to an embodiment of the disclosure.

Referring to FIG. 5, a second electronic device 503 (e.g., the second electronic device 103 of FIG. 1 or the second electronic device 403 of FIG. 4) according to various embodiments may request, from an external electronic device 502, user authentication (OAuth login (ID and password)) for access to an endpoint by using access information, in operation 512. According to various embodiments, when a user clicks address information (e.g., endpoint information or IP address) or a URL, which is received from a first electronic device (e.g., the first electronic device 101 of FIG. 1, the first electronic device 201 of FIG. 2, or the electronic device 401 of FIG. 4), the second electronic device 503 may receive, from the external electronic device 502, a request for an ID and a password, and may provide the ID and the password to the external electronic device 502 so as to request user authentication.

In operation 514, the external electronic device 502 may acquire encryption scheme information included in sharing condition configuration information from a configuration manager 528 included in a login manager (e.g., the login manager 326 of FIG. 3) by using an authentication manager (OAuth manager) 527.

In operation 516, the external electronic device 502 may perform user authentication for the second electronic device 503 by using the ID and the password via the OAuth manager 527 on the basis of the acquired encryption scheme information. According to various embodiments, if the encryption scheme is RSA, and the password uses a secret key, the OAuth manager 527 may decrypt the password by using the ID, so as to perform user authentication for the second electronic device 503. According to various embodiments, the OAuth manager 527 may use an authentication technique (e.g., OAuth standard) related to the user authentication.

In operation 518, after the user authentication succeeds, the external electronic device 502 may acquire encrypted medical information by accessing a storage manager 524 via the OAuth manager 527 (get encrypted EMR data).

In operation 520, the external electronic device 502 may decrypt the encrypted medical information via the OAuth manager 527.

In operation 522, if decryption of the encrypted medical information succeeds, the external electronic device 502 may notify the second electronic device 503 of the success of the user authentication.

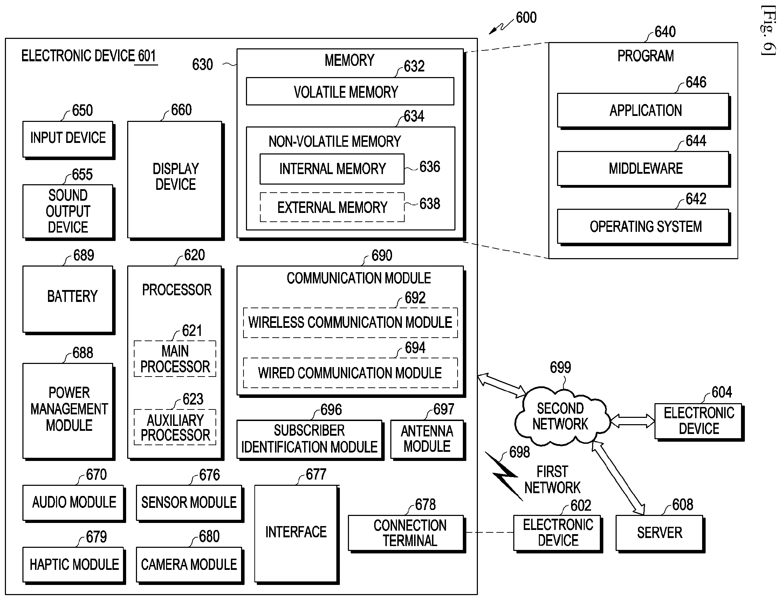

FIG. 6 is a block diagram illustrating an electronic device in a network environment according to an embodiment of the disclosure.

Referring to FIG. 6, the electronic device 601 (e.g., the first electronic device 101 in FIG. 1, the first electronic device 201 in FIG. 2, or the first the electronic device 401 in FIG. 4) in the network environment 600 may communicate with an electronic device 602 via a first network 698 (e.g., a short-range wireless communication network), with or an electronic device 604 or a server 608 via a second network 699 (e.g., a long-range wireless communication network). According to an embodiment, the electronic device 601 may communicate with the electronic device 604 via the server 608. According to an embodiment, the electronic device 601 may include a processor 620, memory 630, an input device 650, a sound output device 655, a display device 660, an audio module 670, a sensor module 676, an interface 677, a haptic module 679, a camera module 680, a power management module 688, a battery 689, a communication module 690, a subscriber identification module 696, or an antenna module 697. In some embodiments, at least one (e.g., the display device 660 or the camera module 680) of the components may be omitted from the electronic device 601, or one or more other components may be added in the electronic device 601. In some embodiments, some of the components may be implemented as single integrated circuitry. For example, the sensor module 676 (e.g., a fingerprint sensor, an iris sensor, or an illuminance sensor) may be implemented as embedded in the display device 660 (e.g., a display).

The processor 620 may execute, for example, software (e.g., a program 640) to control at least one other component (e.g., a hardware or software component) of the electronic device 601 coupled with the processor 620, and may perform various data processing or computation. According to one embodiment, as at least part of the data processing or computation, the processor 620 may load a command or data received from another component (e.g., the sensor module 676 or the communication module 690) in a volatile memory 632, process the command or the data stored in the volatile memory 632, and store resulting data in a non-volatile memory 634. According to an embodiment, the processor 620 may include a main processor 621 (e.g., a central processing unit or an application processor), and an auxiliary processor 623 (e.g., a graphics processing unit, an image signal processor, a sensor hub processor, or a communication processor) that is operable independently from, or in conjunction with, the main processor 621. Additionally or alternatively, the auxiliary processor 623 may be adapted to consume less power than the main processor 621, or to be specific to a specified function. The auxiliary processor 623 may be implemented as separate from, or as part of the main processor 621.

The auxiliary processor 623 may control at least some of functions or states related to at least one component (e.g., the display device 660, the sensor module 676, or the communication module 690) among the components of the electronic device 601, instead of the main processor 621 while the main processor 621 is in an inactive (e.g., sleep) state, or together with the main processor 621 while the main processor 621 is in an active state (e.g., executing an application). According to an embodiment, the auxiliary processor 623 (e.g., an image signal processor or a communication processor) may be implemented as part of another component (e.g., the camera module 680 or the communication module 690) functionally related to the auxiliary processor 623.

The memory 630 may store various data used by at least one component (e.g., the processor 620 or the sensor module 676) of the electronic device 601. The data may include, for example, software (e.g., the program 640) and input data or output data for a command related thereto. The memory 630 may include the volatile memory 632 or the non-volatile memory 634. The non-volatile memory 634 may include internal memory 636 and/or external memory 638.

The program 640 may be stored in the memory 630 as software, and may include, for example, an operating system 642, middleware 644, or an application 646.

The input device 650 may receive a command or data to be used by another component (e.g., the processor 620) of the electronic device 601, from the outside (e.g., a user) of the electronic device 601. The input device 650 may include, for example, a microphone, a mouse, a keyboard, or a digital pen (e.g., a stylus pen).

The sound output device 655 may output sound signals to the outside of the electronic device 601. The sound output device 655 may include, for example, a speaker or a receiver. The speaker may be used for general purposes, such as playing multimedia or playing record, and the receiver may be used for an incoming call. According to an embodiment, the receiver may be implemented as separate from, or as part of the speaker.

The display device 660 may visually provide information to the outside (e.g., a user) of the electronic device 601. The display device 660 may include, for example, a display, a hologram device, or a projector and control circuitry to control a corresponding one of the display, hologram device, and projector. According to an embodiment, the display device 660 may include touch circuitry adapted to detect a touch, or sensor circuitry (e.g., a pressure sensor) adapted to measure the intensity of force incurred by the touch.

The audio module 670 may convert a sound into an electrical signal and vice versa. According to an embodiment, the audio module 670 may obtain the sound via the input device 650, or output the sound via the sound output device 655 or an external electronic device (e.g., an electronic device 602) (e.g., a speaker or a headphone) directly or wirelessly coupled with the electronic device 601.

The sensor module 676 may detect an operational state (e.g., power or temperature) of the electronic device 601 or an environmental state (e.g., a state of a user) external to the electronic device 601, and then generate an electrical signal or data value corresponding to the detected state. According to an embodiment, the sensor module 676 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 677 may support one or more specified protocols to be used for the electronic device 601 to be coupled with the external electronic device (e.g., the electronic device 602) directly or wirelessly. According to an embodiment, the interface 677 may include, for example, a high definition multimedia interface (HDMI), a universal serial bus (USB) interface, a SD card interface, or an audio interface.

A connecting terminal 678 may include a connector via which the electronic device 601 may be physically connected with the external electronic device (e.g., the electronic device 602). According to an embodiment, the connecting terminal 678 may include, for example, a HDMI connector, a USB connector, a SD card connector, or an audio connector (e.g., a headphone connector).

The haptic module 679 may convert an electrical signal into a mechanical stimulus (e.g., a vibration or a movement) or electrical stimulus which may be recognized by a user via his tactile sensation or kinesthetic sensation. According to an embodiment, the haptic module 679 may include, for example, a motor, a piezoelectric element, or an electric stimulator.

The camera module 680 may capture a still image or moving images. According to an embodiment, the camera module 680 may include one or more lenses, image sensors, image signal processors, or flashes.

The power management module 688 may manage power supplied to the electronic device 601. According to one embodiment, the power management module 688 may be implemented as at least part of, for example, a power management integrated circuit (PMIC).

The battery 689 may supply power to at least one component of the electronic device 601. According to an embodiment, the battery 689 may include, for example, a primary cell which is not rechargeable, a secondary cell which is rechargeable, or a fuel cell.

The communication module 690 may support establishing a direct (e.g., wired) communication channel or a wireless communication channel between the electronic device 601 and the external electronic device (e.g., the electronic device 602, the electronic device 604, or the server 608) and performing communication via the established communication channel. The communication module 690 may include one or more communication processors that are operable independently from the processor 620 (e.g., the application processor) and supports a direct (e.g., wired) communication or a wireless communication. According to an embodiment, the communication module 690 may include a wireless communication module 692 (e.g., a cellular communication module, a short-range wireless communication module, or a global navigation satellite system (GNSS) communication module) or a wired communication module 694 (e.g., a local area network (LAN) communication module or a power line communication module). A corresponding one of these communication modules may communicate with the external electronic device via the first network 698 (e.g., a short-range communication network, such as Bluetooth, Wi-Fi direct, or infrared data association (IrDA)) or the second network 699 (e.g., a long-range communication network, such as a cellular network, the Internet, or a computer network (e.g., LAN or WAN). These various types of communication modules may be implemented as a single component (e.g., a single chip), or may be implemented as multi components (e.g., multi chips) separate from each other. The wireless communication module 692 may identify and authenticate the electronic device 601 in a communication network, such as the first network 698 or the second network 699, using subscriber information (e.g., international mobile subscriber identity (IMSI)) stored in the subscriber identification module 696.

The antenna module 697 may transmit or receive a signal or power to or from the outside (e.g., the external electronic device). According to an embodiment, the antenna module may include an antenna including a radiating element composed of a conductive material or a conductive pattern formed in or on a substrate (e.g., PCB). According to an embodiment, the antenna module 697 may include a plurality of antennas. In such a case, at least one antenna appropriate for a communication scheme used in the communication network, such as the first network 698 or the second network 699, may be selected from the plurality of antennas, for example, by the communication module 690. The signal or the power may then be transmitted or received between the communication module 690 and the external electronic device via the selected at least one antenna. According to an embodiment, another component (e.g., a radio-frequency integrated circuit (RFIC)) other than the radiating element may be additionally formed as part of the antenna module 697.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 601 and the external electronic device 604 via the server 608 coupled with the second network 699. Each of the electronic devices 602 and 604 may be a device of a same type as, or a different type, from the electronic device 601. According to an embodiment, all or some of operations to be executed at the electronic device 601 may be executed at one or more of the external electronic devices 602, 604, or 608. For example, if the electronic device 601 should perform a function or a service automatically, or in response to a request from a user or another device, the electronic device 601, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 601. The electronic device 601 may provide the outcome, with or without further processing of the outcome, as at least part of a reply to the request. To that end, a cloud computing, distributed computing, or client-server computing technology may be used, for example.

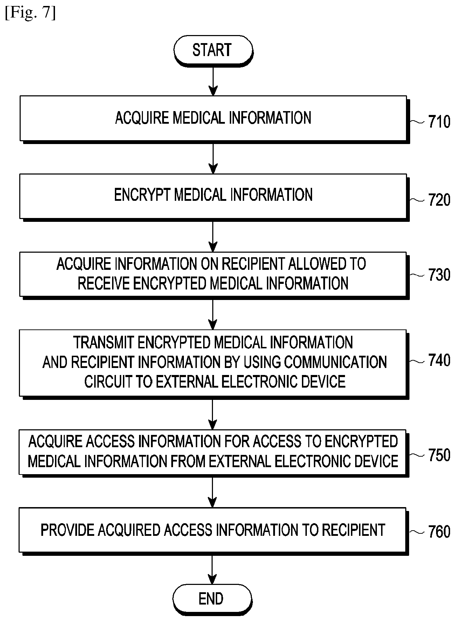

FIG. 7 is a flowchart illustrating a method of sharing medical information of an electronic device according to an embodiment of the disclosure.

Referring to FIG 7, operations 710 to 760 according to various embodiments may be understood as operations performed by a processor (e.g., the processor 220 of FIG. 2 or the processor 620 of FIG. 6. Hereinafter, descriptions will be provided using the processor 620 of FIG. 6 as an example) of a first electronic device (e.g., the first electronic device 201 of FIG. 2, or the electronic device 601 of FIG. 6). According to various embodiments, at least one of operations 710 to 760 may be omitted, a sequence of some operations may be changed, or another operation may be added.

In operation 710, the processor 620 according to various embodiments may temporarily or periodically receive medical information from at least one medical information providing device (e.g., at least one medical information providing device 11-15 of FIG. 1) via a communication circuit (e.g., the communication circuit 210 of FIG. 2 or the communication module 690 of FIG. 6).

In operation 720, the processor 620 according to various embodiments may encrypt the received medical information, and may store the encrypted medical information in a memory (e.g., the memory 230 of FIG. 2 or the memory 630 of FIG. 6). According to various embodiments, the encrypted medical information may be stored in a secured storage area of the memory 630.

In operation 730, the processor 620 according to various embodiments may acquire information of a recipient (e.g., a sharing target for sharing of medical information) allowed to receive the encrypted medical information. According to various embodiments, the recipient allowed to receive the medical information may be a contact selected from at least one contact stored in the memory 630, may be a contact input by a user, or may be a recipient automatically acquired by the processor 620. For example, the recipient allowed to receive the medical information may be included in one of a private group or a public group. The private group may be a group including a recipient that is input by a user or selected by the user from among stored contacts. The public group may be a group including a recipient acquired by the processor 620 on the basis of information, such as a location of a user, a medical activity record of the user, and a public medical institution associated with the user. According to various embodiments, the processor 620 may further acquire sharing condition configuration information of the medical information. According to various embodiments, the sharing condition configuration information of the medical information may include at least one among a sharing (or access) authority, a sharing range, a sharing period, a sharing frequency, an authentication scheme, an authentication validity period, an encryption scheme, and an encryption key of the encrypted medical information. For example, the sharing authority may be an authority allowed to receive the encrypted medical information, the sharing range may be an item allowed to be shared among items included in the encrypted medical information, the sharing period may be a period in which the encrypted medical information may be shared, the sharing frequency may be the number of times that the encrypted medical information may be viewed or received, the authentication scheme may be a user authentication scheme for a recipient, the authentication validity period may be a validity period after user authentication, the encryption scheme may be an encryption scheme of the medical information, and the encryption key may be encryption key information used to decrypt the medical information. According to various embodiments, the sharing condition configuration information may further include various conditions associated with sharing of the encrypted medical information.

In operation 740, the processor 620 according to various embodiments may transmit the encrypted medical information and information of the recipient (e.g., the sharing target for sharing of the medical information) allowed to receive the encrypted medical information to an external electronic device (e.g., the first external electronic device 102 of FIG. 1, the third external electronic device 302 of FIG. 3, the external electronic device 402 of FIG. 4, or the external electronic device 502 of the FIG. 5) by using the communication module 690. The external electronic device 502 may generate (designate) an endpoint (e.g., a storage in which the encrypted medical information is stored) corresponding to the encrypted medical information, and may generate access information enabling access to the endpoint. According to various embodiments, the processor 620 may transmit the sharing condition configuration information of the medical information to the external electronic device 502. The external electronic device 502 may manage the access information and may authenticate a user of the recipient, on the basis of the sharing condition configuration information of the medical information.

In operation 750, the processor 620 according to various embodiments may acquire, from the external electronic device 502, access information for accessing the encrypted medical information. According to various embodiments, the access information may include address information (e.g., endpoint information or IP address) in which the encrypted medical information is stored, or a uniform resource locator (URL) enabling reception of the encrypted medical information.

In operation 760, the processor 620 according to various embodiments may provide the acquired access information to the recipient. According to various embodiments, the processor 620 may transmit the access information to a second electronic device of the recipient (the second electronic device 103 of FIG. 1, the second electronic device 403 of FIG. 4, or the second electronic device 503 of FIG. 5) by means of a text message (e.g., a short message service, an email, or the like). According to various embodiments, the second electronic device 503 may access the endpoint by using the received access information, may receive user authentication by the external electronic device 502, and then may receive medical information of a user of the first electronic device 601.

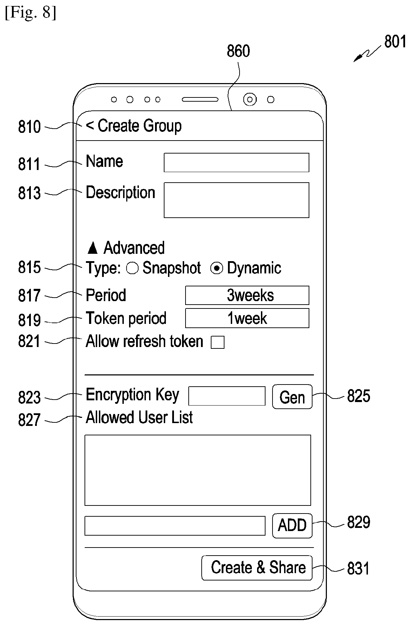

FIG. 8 is an example of a user interface (UI) for sharing medical information of an electronic device according to an embodiment of the disclosure.

Referring to FIG. 8, a processor (e.g., the processor 220 of FIG. 2 or the processor 620 of FIG. 6. Hereinafter, descriptions will be provided using the processor 620 of FIG. 6 as an example) of a first electronic device 801 (e.g., the first electronic device 201 of FIG. 2, the first electronic device 401 of FIG. 4, or the electronic device 601 of FIG. 6) according to various embodiments may display a condition configuration UI screen 810 for sharing medical information, via a display 860 (e.g., the display device 660 of FIG. 6).

According to various embodiments, the processor 620 may display, via the display 860, the condition configuration UI screen 810 for sharing medical information, which includes at least one item for configuring a condition for sharing medical information.

According to various embodiments, the condition configuration UI screen 810 for sharing information may include at least one among a medical information sharing target name (Name) 811, a description of shared medical information (Description) 813, a sharing scheme (Type) 815, a sharing period (Period) 817, an authentication validity period (Token period) 819, a refresh token (Allow refresh token) 821, an encryption key (Encryption key) 823, an encryption key generation icon (Gen) 825, a sharing target 827, a sharing target adding icon (Add) 829, and a generation and sharing icon (Create & Share) 831. In addition, items for other conditions for sharing medical information may further included.

According to various embodiments, the medical information sharing target name (Name) 811 may be a name of a group for sharing of medical information. For example, the medical information sharing target name (Name) 811 may be a name indicating a group for sharing of medical information, such as family, friend, disease name, hospital name, and area name, and may be input by a user.

According to various embodiments, the description of shared medical information (Description) 813 may include description of which medical information is to be shared with whom, and may be input by a user.

According to various embodiments, the sharing scheme (Type) 815 may include whether to share medical information once (Snapshot) or to share medical information multiple times (Dynamic), and may be selected by a user.

According to various embodiments, the sharing period (Period) 817 indicates how long medical information is to be shared, and may be input or selected by a user. For example, if the sharing period (Period) 817 is three weeks, medical information may be shared for three weeks.

According to various embodiments, the authentication validity period (Token period) 819, which is a user authentication validity period of a recipient, may indicate how long user authentication is valid once a user of the recipient is authenticated, and may be input or selected by the user. For example, the authentication validity period (Token period) 819 is one week, the user authentication may be valid for one week with one-time user authentication.

According to various embodiments, the refresh token (Allow refresh token) 821 may indicate whether to update user authentication, and if a user authentication period expires, a user may select whether to enable an update of user authentication.

According to various embodiments, the encryption key (Encryption key) 823 may be an encryption key used for encryption or decryption of medical information or user authentication, and may be generated or input by a user.

According to various embodiments, the encryption key generation icon (Gen) 825 may be an icon for generation of an encryption key used for encryption or decryption of medical information or user authentication. If a user selects (or clicks) the encryption key generation icon (Gen) 825, an encryption key may be generated, and may be input to or displayed in "Encryption key" 823.

According to various embodiments, the sharing target 827 may include a recipient allowed to receive medical information of a user. For example, the sharing target 827 may include one or more contacts. For example, the sharing target 827 may be selected from one of a private group and a public group, so as to be included. The private group may be a group including a recipient that is input by a user or selected by the user from among stored contacts. The public group may be a group including a recipient acquired on the basis of information, such as a location of a user, a medical activity record of the user, and a public medical institution associated with the user.

According to various embodiments, the sharing target adding icon (Add) 829 may be an icon for adding a sharing target to the sharing target 827.

According to various embodiments, the generation and sharing icon (Create & Share) 831 may be an icon for generation of a condition configuration for sharing medical information, and storing the generated condition configuration information.

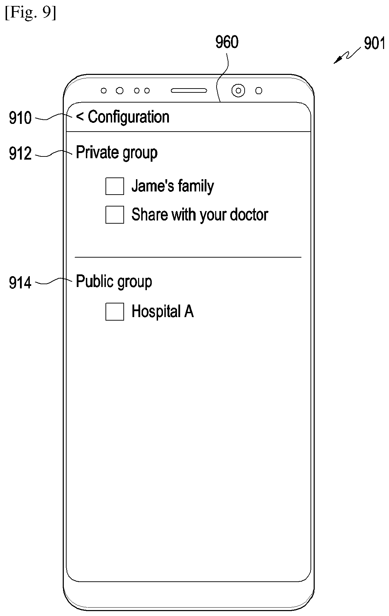

FIG. 9 is an example of a screen for groups subject to sharing of medical information according to an embodiment of the disclosure.

Referring to FIG. 9, a processor (e.g., the processor 220 of FIG. 2 or the processor 620 of FIG. 6. Hereinafter, descriptions will be provided using the processor 620 of FIG. 6 as an example) of a first electronic device 901 (e.g., the first electronic device 201 of FIG. 2, the first electronic device 401 of FIG. 4, or the electronic device 601 of FIG. 6) according to various embodiments may display a medical information sharing target group screen 910 on a display 960 (e.g., the display device 660 of FIG. 6).

According to various embodiments, the processor 620 may display, via the display 960, the medical information sharing target group screen 910 including a private group 912 and a public group 914. For example, the private group may be a group including a recipient that is input by a user or selected by the user from among stored contacts, and may be a group including James family, as shown in "James family", or may be a group including the user's doctor, as shown in "share with your doctor". The public group may be a group including a recipient acquired on the basis of information, such as a location of a user, a medical activity record of the user, and a public medical institution associated with the user, and may be a hospital visited by the user, as shown in "Hospital A" or may be a hospital located near the user. According to various embodiments, the user may select a recipient group included in the private group 912 or the public group 914.

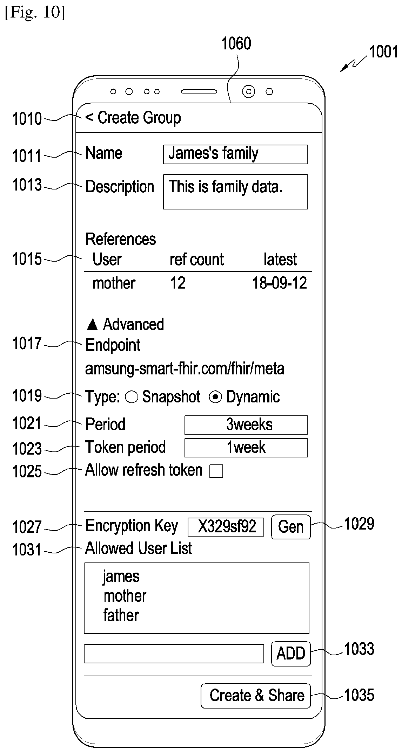

FIG. 10 is an example illustrating a screen for, when medical information is being shared, the medical information that is being shared, according to an embodiment of the disclosure.

Referring to FIG. 10, a processor (e.g., the processor 220 of FIG. 2 or the processor 620 of FIG. 6. Hereinafter, descriptions will be provided using the processor 620 of FIG. 6 as an example) of a first electronic device 1001 (e.g., the first electronic device 201 of FIG. 2, the first electronic device 401 of FIG. 4, or the electronic device 601 of FIG. 6) according to various embodiments may display a screen 1010 for medical information that is being shared, via a display 1060 (e.g., the display device 660 of FIG. 6).

According to various embodiments, if the medical information is being shared, the screen 1010 for medical information that is being shared may include at least one among a medical information sharing target name (Name) 1011, a description of shared medical information (Description) 1013, a shared medical information viewing record (References) 1015, an endpoint (Endpoint) 1017, a sharing scheme (Type) 1019, a sharing period (Period) 1021, an authentication validity period (Token period) 1023, a refresh token (Allow refresh token) 1025, an encryption key (Encryption key) 1027, an encryption key generation icon (Gen) 1029, a sharing target 1031, a sharing target adding icon (Add) 1033, and a generation and sharing icon (Create & Share) 1035. In addition, other items relating to medical information that is being shared may be further included.

According to various embodiments, the screen 1010 for medical information that is being shared may indicate that medical information named "James family" is being shared with "James", "mother", and "father" by using an endpoint of "amsung-smart-fhir.com/fhir/meta", a medical information sharing period is three weeks, one time user authentication is valid for one week, and an encryption key is "x329sf92". A user may check, via the screen 1010 for medical information that is being shared, which medical information is shared with whom by means of which encryption key via which endpoint in which scheme, and may check who has viewed the medical information.

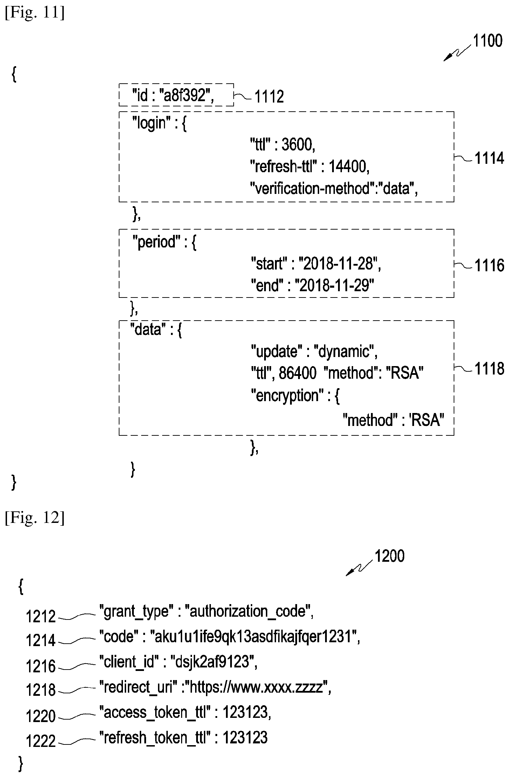

FIG. 11 is a diagram illustrating an example of data representing medical information sharing condition configuration information according to an embodiment of the disclosure.

Referring to FIG. 11, a processor (e.g., the processor 220 of FIG. 2 or the processor 620 of FIG. 6. Hereinafter, descriptions will be provided using the processor 620 of FIG. 6 as an example) of a first electronic device (e.g., the first electronic device 201 of FIG. 2, the electronic device 401 of FIG. 4, the electronic device 601 of FIG. 6, the electronic device 801 of FIG. 8, the electronic device 901 of FIG. 9, or the electronic device 1001 of FIG. 10) according to various embodiments may generate and store data 1100 relating to condition configuration information for sharing of medical information (e.g., json, xml, or the like), and may transfer the generated data 1100 to an external electronic device (e.g., the external electronic device 102 of FIG. 1, the external electronic device 302 of FIG. 3, or the electronic device 402 of FIG. 4).

For example, the data 1100 relating to the condition configuration information for sharing of the medical information may include at least one of an identity (ID) item 1112, a login item 1114, a period item 1116, and a data item 1118. The ID item 1112 may include ID (e.g., identity information), and the login item 1114 may include information for authentication and information on an authentication scheme. The period item 1116 may include a sharing period, the data item 1118 may include a sharing scheme, an encryption scheme, and/or information relating to whether to perform update.

An external electronic device (e.g., the external electronic device 102 of FIG. 1, the external electronic device 302 of FIG. 3, or the electronic device 402 of FIG. 4) may authenticate a recipient allowed to share medical information and may allow the medical information to be provided to the authenticated recipient on the basis of data 1100 relating to condition configuration information for sharing of the medical information.

FIG. 12 is an example of an authentication message according to an embodiment of the disclosure.

Referring to FIG. 12, an external electronic device (e.g., the external electronic device 102 of FIG. 1, the external electronic device 302 of FIG. 3, or the electronic device 402 of FIG. 4) may use a standard authentication message 1200, for example, an OAuth login response message, for user authentication of a recipient.

According to various embodiments, the OAuth login response message 1200 may include grant_type 1212, code 1214, client_id 1216, redirect_uri 1218, access token ttl 1220, and refresh token ttl 1222. For example, grant_type 1212 may include authentication scheme information, code 1214 may include authentication code information according to the authentication scheme, client_id 1216 may include an id of a recipient user, redirect_uri 1218 may include endpoint information, access_token_ttl 1220 may include login validity time information, and refresh_token_ttl 1222 may include information on whether to update a validity time.

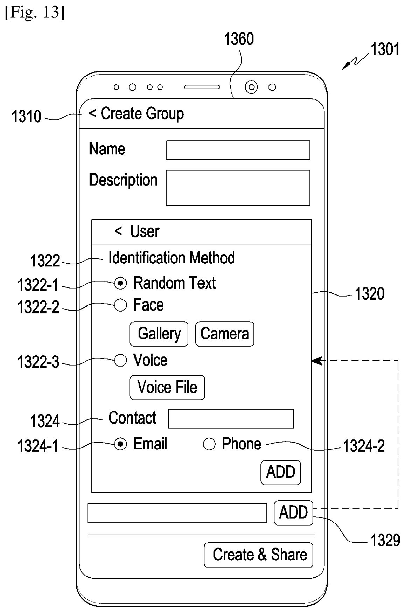

FIG. 13 is an example of a screen for a user authentication scheme configuration according to an embodiment of the disclosure.

Referring to FIG. 13, a processor (e.g., the processor 220 of FIG. 2 or the processor 620 of FIG. 6. Hereinafter, descriptions will be provided using the processor 620 of FIG. 6 as an example) of a first electronic device 1301 (e.g., the first electronic device 201 of FIG. 2, the electronic device 401 of FIG. 4, the electronic device 601 of FIG. 6, the electronic device 801 of FIG. 8, the electronic device 901 of FIG. 9, or the electronic device 1001 of FIG. 10) according to various embodiments may display a user authentication scheme configuration screen 1320 corresponding to a recipient via a display 1360.

According to various embodiments, the processor 620 may display, a screen 1310 for medical information that is being shared, via the display 1360, the user authentication scheme configuration screen 1320, via which an authentication scheme (Identification Method) 1322 and a contact 1324 can be selected. For example, if an additional icon 1329 is selected on the user authentication scheme configuration screen 1320, the processor 620 may display the user authentication scheme configuration screen 1320.

According to various embodiments, the authentication scheme 1322 may be able to select one of Random Text 1322-1, Face 1322-2, and Voice 1322-3. Random Text 1322-1 may be a scheme of authenticating a recipient by using a random text, such as a random password. If random text, to which a recipient is designated, is input, authentication of a user of the recipient may be successful. Face 1322-2 may be a scheme of authenticating a recipient by using an image of the recipient, which is obtained via a gallery and a camera. If a registered image of the recipient and an image received from a recipient user are the same, user authentication of the recipient may be successful. Voice 1322-3 may be a scheme of authenticating a recipient by using a voice file corresponding to a voice of the recipient. If the voice file and a voice received from a recipient user are the same, user authentication of the recipient may be successful. According to various embodiments, other authentication schemes may be possible for the authentication scheme 1322, in addition to Random Text 1322-1, Face 1322-2, and Voice 1322-3. For example, an authentication scheme using biometric information (fingerprint, iris, face, voice, vein, or the like) of a recipient, other than face, may be possible.

It may be possible to select one of Email 1324-1 and Phone 1324-2, for contact 1324. The selected authentication scheme and contact may be input as sharing configuration information.

Each of elements described in the document may include one or more components, and a name of a corresponding element may vary according to a type of an electronic device. In various embodiments, an electronic device may include at least one of the elements described in the document, wherein some elements are omitted, or additional other elements are further included. One entity is configured by combining some elements of the electronic device, and therefore functions of the elements before the combination may be performed in the same manner.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101). For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStoreTM), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

According to various embodiments, in a storage medium that stores commands, the commands are configured, when executed by at least one circuit, to cause the at least one circuit to perform at least one operation, and the at least one operation includes: acquiring medical information; encrypting the medical information; acquiring information of at least one recipient allowed to receive the encrypted medical information; transmitting, to an external electronic device, the encrypted medical information and information of the at least one recipient by means of the communication circuit; acquiring, from the external electronic device, access information for accessing the encrypted medical information; and providing the acquired access information to the at least one recipient.

A computer-readable recording medium may include a hard disk, a floppy disk, magnetic media (e.g., a magnetic tape), optical media (e.g., compact disc read only memory (CD-ROM) and digital versatile disc (DVD)), magneto-optical media (e.g., a floptical disk), a hardware device (e.g., read only memory (ROM), random access memory (RAM), or flash memory), etc. A program command may include a high-level language code executable by a computer using an interpreter, etc., as well as a machine language code produced by a compiler. The hardware device described above may be configured to operate as one or more software modules to perform operations of the various embodiments, and vice versa.

The electronic device of the various embodiments described above is not limited to the above-described embodiments and drawings, and may be variously substituted, modified, and changed.

## Claims

1. An electronic device (201, 401), comprising:
a communication circuit (210);
a memory (230) configured to store instructions; and
at least one processor (220), wherein the instructions are configured, when executed by the at least one processor (220), to cause the electronic device (201, 401) to:
receive medical information from a medical information providing device (404);
encrypt the medical information;
transmit, to an external electronic device (402) via the communication circuit (210), the encrypted medical information and information of at least one recipient authorized to receive the encrypted medical information, the recipient being a user of a second electronic device (403);
obtain, from the external electronic device (402), access information which enables access to the encrypted medical information; and
provide the obtained access information and decryption information for decryption of the encrypted medical information to the second electronic device (403).

2. The electronic device (201, 401) of claim 1, wherein the access information comprises address information in which the encrypted medical information is stored.

3. The electronic device (201, 401) of claim 1, wherein the access information comprises a uniform resource locator (URL) enabling reception of the encrypted medical information.

4. The electronic device (201, 401) of claim 1, wherein the access information is provided to the at least one recipient using a message (1200).

5. The electronic device (201, 401) of claim 1, wherein the instructions are configured to cause the electronic device (201, 401) to provide a sharing condition associated with the medical information to the external electronic device (402).

6. The electronic device (201, 401) of claim 1, wherein the instructions are configured to cause the electronic device (201, 401) to configure a sharing condition comprising at least one of an authority, a sharing range, a sharing period, a sharing frequency, a user authentication scheme (1322), an authentication validity period, an encryption scheme, or an encryption key, which is associated with the medical information.

7. The electronic device (801) of claim 6, further comprising:
a display (860),
wherein the instructions are configured to cause the electronic device (201, 401) to display, on the display (860), a first screen for configuration of the sharing condition.

8. The electronic device (1001) of claim 6,
wherein the instructions are configured to cause the electronic device (201, 401) to display, on the display (1060), a second screen for selection of the user authentication scheme (1322).

9. The electronic device (1301) of claim 8, wherein the user authentication scheme (1322) comprises at least one of a random text (1322-1) scheme, an iris recognition scheme, a fingerprint recognition scheme, a face (1322-2) recognition scheme, a voice (1322-3) recognition scheme, or a vein recognition scheme.

10. The electronic device (201, 401) of claim 1,
wherein the memory (230) comprises a normal storage area and a secured storage area, and
wherein the received medical information or the encrypted medical information is stored in the secured storage area.

11. A method for sharing medical information by an electronic device (201, 401), the method comprising:
receiving (417) medical information from a medical information providing device (404);
encrypting the medical information;
transmitting (423) the encrypted medical information and information of at least one recipient authorized to receive the encrypted medical information, the at least one recipient being a user of a second electronic device to an external electronic device (402);
obtaining (425) access information which enables access to the encrypted medical information from the external electronic device (402); and
providing (431) the obtained access information and decryption information for decryption of the encrypted medical information to second electronic device (403).

12. A non-transitory computer-readable recording medium having recorded thereon at least one program comprising commands, which, when executed by an electronic device (201,401) performs a method, the method comprising:
receiving (417) medical information from a medical information providing device (404);
encrypting the medical information;
transmitting (423), to an external electronic device (402), the encrypted medical information and information of at least one recipient authorized to receive the encrypted medical information, the recipient being a user of a second electronic device (403);
obtaining (425), from the external electronic device (402), access information which enables access to the encrypted medical information; and
providing (431) the obtained access information and decryption information for decryption of the encrypted medical information to the second electronic device (403).

## Patentansprüche

1. Elektronische Vorrichtung (201, 401), umfassend:
eine Kommunikationsschaltung (210);
einen Speicher (230), der zum Speichern von Anweisungen konfiguriert ist; und
mindestens einen Prozessor (220), wobei die Anweisungen so konfiguriert sind, dass sie, wenn sie von dem mindestens einen Prozessor (220) ausgeführt werden, die elektronische Vorrichtung (201, 401) dazu veranlassen:
medizinische Informationen von einer Vorrichtung (404) zur Bereitstellung medizinischer Informationen zu empfangen;
die medizinischen Informationen zu verschlüsseln;
die verschlüsselten medizinischen Informationen und Informationen über mindestens einen Empfänger, der berechtigt ist, die verschlüsselten medizinischen Informationen zu empfangen, über die Kommunikationsschaltung (210) an eine externe elektronische Vorrichtung (402) zu übertragen, wobei der Empfänger ein Benutzer einer zweiten elektronischen Vorrichtung (403) ist;
von der externen elektronischen Vorrichtung (402) Zugangsinformationen zu erhalten, die den Zugriff auf die verschlüsselten medizinischen Informationen ermöglichen; und
die erhaltenen Zugangsinformationen und Entschlüsselungsinformationen für die Entschlüsselung der verschlüsselten medizinischen Informationen an die zweite elektronische Vorrichtung (403) zu liefern.

2. Elektronische Vorrichtung (201, 401) nach Anspruch 1, wobei die Zugangsinformationen Adressinformationen umfassen, in denen die verschlüsselten medizinischen Informationen gespeichert sind.

3. Elektronische Vorrichtung (201, 401) nach Anspruch 1, wobei die Zugangsinformationen einen Uniform Resource Locator (URL) umfassen, der den Empfang der verschlüsselten medizinischen Informationen ermöglicht.

4. Elektronische Vorrichtung (201, 401) nach Anspruch 1, wobei die Zugangsinformationen dem mindestens einen Empfänger unter Verwendung einer Nachricht (1200) bereitgestellt werden.

5. Elektronische Vorrichtung (201, 401) nach Anspruch 1, wobei die Anweisungen so konfiguriert sind, dass sie die elektronische Vorrichtung (201, 401) veranlassen, eine mit den medizinischen Informationen verbundene Teilungsbedingung an die externe elektronische Vorrichtung (402) zu liefern.

6. Elektronische Vorrichtung (201, 401) nach Anspruch 1, wobei die Anweisungen so konfiguriert sind, dass sie die elektronische Vorrichtung (201, 401) veranlassen, eine Teilungsbedingung zu konfigurieren, die mindestens eines einer Autorität, eines Teilungsbereichs, einer Teilungsperiode, einer Teilungshäufigkeit, eines Benutzerauthentifizierungsschemas (1322), einer Authentifizierungsgültigkeitsperiode, eines Verschlüsselungsschemas oder eines Verschlüsselungsschlüssels, der mit den medizinischen Informationen verbunden ist, umfasst.

7. Elektronische Vorrichtung (801) nach Anspruch 6, die ferner umfasst:
eine Anzeige (860),
wobei die Anweisungen so konfiguriert sind, dass sie die elektronische Vorrichtung (201, 401) veranlassen, auf der Anzeige (860) einen ersten Bildschirm zur Konfiguration der Teilungsbedingung anzuzeigen.

8. Elektronische Vorrichtung (1001) nach Anspruch 6,
wobei die Anweisungen so konfiguriert sind, dass sie die elektronische Vorrichtung (201, 401) veranlassen, auf der Anzeige (1060) einen zweiten Bildschirm zur Auswahl des Benutzerauthentifizierungsschemas (1322) anzuzeigen.

9. Elektronische Vorrichtung (1301) nach Anspruch 8, wobei das Benutzerauthentifizierungsschema (1322) mindestens eines eines Zufallstext-(1322-1)-Schemas, eines Iriserkennungsschemas, eines Fingerabdruckerkennungsschemas, eines Gesichts-(1322-2)-Erkennungsschemas, eines Stimm-(1322-3)-Erkennungsschemas oder eines Venenerkennungsschemas umfasst.

10. Elektronische Vorrichtung (201, 401) nach Anspruch 1,
wobei der Speicher (230) einen normalen Speicherbereich und einen gesicherten Speicherbereich umfasst, und
wobei die empfangenen medizinischen Informationen oder die verschlüsselten medizinischen Informationen in dem gesicherten Speicherbereich gespeichert werden.

11. Verfahren zum Teilen medizinischer Informationen durch eine elektronische Vorrichtung (201, 401), wobei das Verfahren Folgendes umfasst:
Empfangen (417) medizinischer Informationen von einer Vorrichtung (404) zur Bereitstellung medizinischer Informationen;
Verschlüsseln der medizinischen Informationen;
Übertragen (423) der verschlüsselten medizinischen Informationen und der Informationen mindestens eines Empfängers, der zum Empfangen der verschlüsselten medizinischen Informationen berechtigt ist, wobei der mindestens eine Empfänger ein Benutzer einer zweiten elektronischen Vorrichtung ist, an eine externe elektronische Vorrichtung (402);
Erhalten (425) von Zugangsinformationen, die den Zugriff auf die verschlüsselten medizinischen Informationen ermöglichen, von der externen elektronischen Vorrichtung (402); und
Liefern (431) der erhaltenen Zugangsinformationen und Entschlüsselungsinformationen zur Entschlüsselung der verschlüsselten medizinischen Informationen an die zweite elektronische Vorrichtung (403).

12. Nicht-flüchtiges, computerlesbares Aufzeichnungsmedium, auf dem mindestens ein Programm aufgezeichnet ist, das Befehle umfasst, die, wenn sie von einer elektronischen Vorrichtung (201, 401) ausgeführt werden, ein Verfahren durchführen, wobei das Verfahren Folgendes umfasst:
Empfangen (417) medizinischer Informationen von einer Vorrichtung (404) zur Bereitstellung medizinischer Informationen;
Verschlüsseln der medizinischen Informationen;
Übertragen (423) der verschlüsselten medizinischen Informationen und von Informationen mindestens eines Empfängers, der zum Empfangen der verschlüsselten medizinischen Informationen berechtigt ist, an eine externe elektronische Vorrichtung (402), wobei der Empfänger ein Benutzer einer zweiten elektronischen Vorrichtung (403) ist;
Erhalten (425), von der externen elektronischen Vorrichtung (402), von Zugangsinformationen, die den Zugriff auf die verschlüsselten medizinischen Informationen ermöglichen; und
Liefern (431) der erhaltenen Zugangsinformationen und Entschlüsselungsinformationen zur Entschlüsselung der verschlüsselten medizinischen Informationen an die zweite elektronische Vorrichtung (403).

## Revendications

1. Dispositif électronique (201, 401), comprenant :
un circuit de communication (210) ;
une mémoire (230) configurée pour stocker des instructions ; et
au moins un processeur (220), dans lequel les instructions sont configurées, lorsqu'elles sont exécutées par l'au moins un processeur (220), pour amener le dispositif électronique (201, 401) à :
recevoir des informations médicales d'un dispositif de fourniture d'informations médicales (404) ;
crypter les informations médicales ;
transmettre, à un dispositif électronique externe (402) via le circuit de communication (210), les informations médicales cryptées et les informations d'au moins un destinataire autorisé à recevoir les informations médicales cryptées, le destinataire étant un utilisateur d'un deuxième dispositif électronique (403) ;
obtenir, à partir du dispositif électronique externe (402), des informations d'accès permettant d'accéder aux informations médicales cryptées ; et
fournir les informations d'accès obtenues et les informations de décryptage pour le décryptage des informations médicales cryptées au deuxième dispositif électronique (403) .

2. Dispositif électronique (201, 401) de la revendication 1, dans lequel les informations d'accès comprennent des informations d'adresse dans lesquelles les informations médicales cryptées sont stockées.

3. Dispositif électronique (201, 401) de la revendication 1, dans lequel les informations d'accès comprennent un localisateur de ressources uniformes (URL) permettant la réception des informations médicales cryptées.

4. Dispositif électronique (201, 401) de la revendication 1, dans lequel les informations d'accès sont fournies à l'au moins un destinataire au moyen d'un message (1200).

5. Dispositif électronique (201, 401) de la revendication 1, dans lequel les instructions sont configurées pour amener le dispositif électronique (201, 401) à fournir une condition de partage associée aux informations médicales au dispositif électronique externe (402).

6. Dispositif électronique (201, 401) de la revendication 1, dans lequel les instructions sont configurées pour amener le dispositif électronique (201, 401) à configurer une condition de partage comprenant au moins un d'une autorité, d'une plage de partage, d'une période de partage, d'une fréquence de partage, d'un schéma d'authentification d'utilisateur (1322), d'une période de validité d'authentification, d'un schéma de cryptage ou d'une clé de cryptage, qui est associée à l'information médicale.

7. Dispositif électronique (801) de la revendication 6, comprenant en outre :
un affichage (860),
dans lequel les instructions sont configurées pour amener le dispositif électronique (201, 401) à afficher, sur l'affichage (860), un premier écran pour configurer la condition de partage.

8. Dispositif électronique (1001) de la revendication 6,
dans lequel les instructions sont configurées pour amener le dispositif électronique (201, 401) à afficher, sur l'écran (1060), un deuxième écran pour la sélection du schéma d'authentification d'utilisateur (1322).

9. Dispositif électronique (1301) de la revendication 8, dans lequel le schéma d'authentification d'utilisateur (1322) comprend au moins un d'un schéma de texte aléatoire (1322-1), d'un schéma de reconnaissance 'iris, d'un schéma de reconnaissance d'empreintes digitales, d'un schéma de reconnaissance de visage (1322-2), d'un schéma de reconnaissance de voix (1322-3) ou d'un schéma de reconnaissance de veines.

10. Dispositif électronique (201, 401) de la revendication 1,
dans lequel la mémoire (230) comprend une zone de stockage normale et une zone de stockage sécurisée, et
dans lequel les informations médicales reçues ou les informations médicales cryptées sont stockées dans la zone de stockage sécurisée.

11. Procédé de partage d'informations médicales par un dispositif électronique (201, 401), le procédé comprenant :
recevoir (417) des informations médicales provenant d'un dispositif de fourniture d'informations médicales (404) ;
crypter les informations médicales ;
transmettre (423) les informations médicales cryptées et les informations d'au moins un destinataire autorisé à recevoir les informations médicales cryptées, l'au moins un destinataire étant un utilisateur d'un deuxième dispositif électronique vers un dispositif électronique externe (402) ;
obtenir (425) des informations d'accès permettant d'accéder aux informations médicales cryptées à partir du dispositif électronique externe (402) ; et
fournir (431) les informations d'accès obtenues et les informations de décryptage pour le décryptage des informations médicales cryptées au deuxième dispositif électronique (403).

12. Support d'enregistrement non transitoire lisible par ordinateur sur lequel est enregistré au moins un programme comprenant des commandes qui, lorsqu'elles sont exécutées par un dispositif électronique (201, 401), metten en oeuvre un procédé, le procédé comprenant :
recevoir (417) des informations médicales provenant d'un dispositif de fourniture d'informations médicales (404) ;
crypter les informations médicales ;
transmettre (423), à un dispositif électronique externe (402), les informations médicales cryptées et les informations d'au moins un destinataire autorisé à recevoir les informations médicales cryptées, le destinataire étant un utilisateur d'un deuxième dispositif électronique (403) ;
obtenir (425), à partir du dispositif électronique externe (402), des informations d'accès permettant d'accéder aux informations médicales cryptées ; et
fournir (431) les informations d'accès obtenues et les informations de décryptage pour le décryptage des informations médicales cryptées au deuxième dispositif électronique (403).
